Europäisches Patentamt

**European Patent Office**

Office européen des brevets

(11) Numéro de publication: **0 086 143
B1**

(12) **FASCICULE DE BREVET EUROPEEN**

(45) Date de publication du fascicule du brevet:
04.06.86

(21) Numéro de dépôt: **83400207.3**

(22) Date de dépôt: **31.01.83**

(51) Int. Cl.⁴: **G 01 N 21/90**

(54) Procédé et dispositif de détection de corps étrangers dans un liquide.

(30) Priorité: **01.02.82 FR 8201542**

(43) Date de publication de la demande:
**17.08.83 Bulletin 83/33**

(45) Mention de la délivrance du brevet:
**04.06.86 Bulletin 86/23**

(84) Etats contractants désignés:
**AT BE CH DE FR GB IT LI LU NL SE**

(56) Documents cités:
**FR - A - 2 284 877
FR - A - 2 390 709
FR - A - 2 486 241
US - A - 3 598 907
US - A - 3 777 169
US - A - 3 818 127
US - A - 3 830 969
US - A - 4 136 930**

(73) Titulaire: **AEROSPATIALE Société Nationale Industrielle, 37 boulevard de Montmorency, F-75781 Paris Cédex 16 (FR)**
Titulaire: **SANOFI, société anonyme, 40, Avenue George V, F-75008 Paris (FR)**

(72) Inventeur: **Ometz, Pierre Henri Maurice, 7 Villepreux Village, F-33160 Saint-Aubin-de Medoc (FR)**
Inventeur: **Labrador Jacques Alphonse Louis, 24 rue du Jeu de Paume, F-33200 Bordeaux-Cauderan (FR)**

(74) Mandataire: **Lepeudry-Gautherat, Thérèse et al, ARMENGAUD JEUNE CABINET LEPEUDRY 23 boulevard de Strasbourg, F-75010 Paris (FR)**

ACTORUM AG

# Description

La présente invention concerne un procédé et un dispositif de détection de corps étrangers, notamment solides, dans un liquide, et a plus particulièrement pour objet de tels procédé et dispositif permettant une détection automatique, rapide et fiable des corps étrangers, tels que des particules solides ou des défauts, présents dans un liquide contenu dans un récipient, transparent au mode de détection, et indésirables au-dessus d'une taille apparente prédéterminée, quelles que soient leur forme et leur nature.

L'invention se rapporte donc, d'une manière générale, aux procédé et dispositif de contrôle non destructif, susceptible d'être effectué même après fermeture définitive du récipient, en vue de la commercialisation de ce dernier, et, dans la suite de la présente description, l'invention sera exposée en référence au contrôle non destructif de fabrications pharmaceutiques telles que des ampoules et/ou flacons de produits injectables, sans être toutefois limitée à cette application, qui n'est donnée qu'à titre d'exemple particulier.

On sait, en effet, que les liquides injectables présentés en ampoules et/ou flacons doivent être soumis à des contrôles rigoureux, ayant notamment pour objet de détecter, après scellement des ampoules ou flacons, la présence de défauts de nature, de forme et de tailles très diverses: filaments textiles (provenant des vêtements, des filtres, de l'atmosphère) particules granulaires, particules de verre ou résultant d'une cristallisation, poussière, etc... Le but de cette détection est de permettre l'élimination des récipients contenant des défauts inadmissibles.

A cet effet, il a déjà été proposé de contrôler des ampoules de produits injectables par la mise en œuvre d'un procédé consistant à mettre une ampoule en mouvement, par exemple à l'entraîner en rotation sur elle-même, autour de son axe longitudinal, puis à l'immobiliser brusquement, afin de développer dans l'ampoule immobilisée un mouvement sensiblement circulaire de son contenu.

La surface utile de l'ampoule définie par sa largeur (correspondant à son diamètre, dans la plupart des cas) et la hauteur du liquide qu'elle contient, est ensuite explorée par une caméra de télévision classique, de type analogique, et des images successives ainsi obtenues sont comparées, ce qui permet de détecter la présence des particules contenues dans le liquide, en réduisant les risques d'erreurs liés aux éventuelles sources de signal parasite, immobiles et constantes, comme par exemple un défaut dans la matière dans laquelle est constituée l'ampoule, et/ou une impression portée sur l'ampoule.

Mais les micro-instabilités de lecture des images, inhérentes au balayage analogique, sont la cause d'un fonctionnement médiocre, car la position de la zone explorée à chaque instant est déterminée par deux tensions électriques variables, définissant l'une la position horizontale (abscisse) et l'autre la position verticale (ordonnée). Comme ces tensions sont continuellement variables, et que chaque point de l'image n'est pas physiquement ou mécaniquement délimité, il en résulte une légère différence apparente de position de l'image d'un même point, d'une lecture à l'autre, et donc qu'un objet fixe, telle qu'une lettre ou partie de lettre imprimée sur l'ampoule, est considéré comme mobile et par conséquent assimilé à un corps étranger, lorsque deux images successives sont comparées entre elles.

Un tel dispositif de détection crée donc, de lui-même, des défauts factices, et conduit à de nombreuses erreurs de détection, ce qui en rend l'utilisation incompatible avec la fiabilité exigée en particulier dans le domaine pharmaceutique.

Il est par ailleurs connu, par nos demandes de brevets FR-A 2 390 709 et 2 451 043, de détecter la présence de matières étrangères dans des liquides au moyen de procédés et d'appareils permettant d'explorer une zone de faible superficie de l'ampoule. L'ampoule, immobilisée et dont le contenu est entraîné selon un mouvement pseudo-circulaire, comme expliqué ci-dessus, est interposée entre une source de lumière, pour laquelle l'ampoule est transparente, et un dispositif photo-détecteur, l'axe de la source de lumière étant commun ou au moins très proche et sensiblement parallèle à l'axe optique du dispositif photo-détecteur. La variation de lumière, résultant du passage devant cette zone de faible superficie d'une particule étrangère en suspension dans le liquide, et entraînée en rotation avec ce dernier dans son mouvement pseudo-circulaire, permet de la détecter. Ce procédé de détection par occultation, puisque des corps étrangers et défauts occultent la lumière incidente et créent une diminution de la lumière transmise reçue par le dispositif photodétecteur, est également un procédé de détection en continu, puisque pendant le temps du contrôle, le dispositif photodétecteur ne cesse de surveiller l'ampoule.

Dans la demande de brevet français FR-A 2 451 043, la surface photosensible du dispositif photo-détecteur et constituée par une pluralité de petits récepteurs de lumière, alignés selon une réglette parallèle à une génératrice de l'ampoule.

Dans l'autre demande de brevet français précitée, FR-A 2 390 709, la surface photosensible du dispositif photo-détecteur est divisée en une multiplicité de régions microphoto-réceptrices, qui sont disposées en une ou en quelques colonnes parallèles à l'axe de l'ampoule, avec ou sans chevauchement dans la direction perpendiculaire à l'axe de l'ampoule dans ce dernier cas, et, éventuellement de plus, en une ou quelques lignes parallèles à la direction perpendiculaire à l'axe de l'ampoule, au niveau de la projection du fond de cette dernière. Sur la surface photosensible, composée de vingt à cent vingt éléments distincts, mesurant chacun de 0,01 à 1 mm², la superficie relative du microphoto-récepteur est rendue variable en fonction des limites de détection. Le champ de contrôle peut être modifié en fonction de la taille du récipient transparent, en modifiant le nombre de microphoto-récepteurs utilisés au moyen d'un circuit de sélection. Un commutateur permet d'utiliser un nombre plus ou moins grand de microphoto-récepteurs, disposés en une ou plusieurs rangées verticales, dont la dimension verticale est fonction de la dimension de la plus grande ampoule devant pouvoir être contrôlée, et éventuellement disposés également en une ou plusieurs rangées horizontales, dont la dimension est fonction de la largeur de la plus large des ampoules devant pouvoir être contrôlée, cette ou ces rangées horizontales étant disposées de façon à correspondre à la base des ampoules.

Dans ces diverses réalisations, chaque élément photo-récepteur est constitué d'un faisceau de fibres optiques aboutissant contre un élément convertisseur photo-électrique, tel qu'une photo-diode, un photo-transistor ou toute autre cellule photo-électrique convenable, connectée à au moins un amplificateur, qui lui est propre, dans un circuit analogique de détection de particule, comprenant un condensateur, éliminant la composante continue du signal, et un comparateur à seuil, dont le seuil correspond à une valeur standard, de référence, préalablement fixée et établie pour le niveau de détection des particules étrangères. Un circuit «OU» rassemble ensuite les signaux provenant des différents comparateurs à seuil, et commande l'élimination de l'ampoule si l'un de ces derniers délivre un signal de dépassement de seuil.

Dans le meilleur des cas, la surface surveillée par ces dispositifs connus correspond à une zone de contrôle en forme de T renversé, dont une barre, parallèle à l'axe de l'ampoule, correspond à une génératrice ou à une mince bande sur l'ampoule, de largeur très inférieure à celle de l'ampoule, et dont l'autre barre, perpendiculaire à l'axe de l'ampoule, correspond à une mince bande horizontale contre la base de l'ampoule, dont elle couvre le diamètre, et de hauteur très inférieure à celle du liquide de l'ampoule.

Ces dispositifs ne permettent pas d'obtenir la fiabilité requise dans le contrôle des fabrications pharmaceutiques, car la surveillance limitée à une très faible proportion de la surface apparente du liquide, et en général à une génératrice plus ou moins large restant fixe mais toujours très inférieure à la largeur de l'ampoule, rend difficile le contrôle d'ampoules marquées ou imprimées. En effet, il arrive fréquemment que l'impression figurant sur l'ampoule occulte une fraction importante de la petite zone surveillée, et qu'une particule en suspension ne puisse être détectée.

Il est connu également par les brevets US-A 3 777 169 et 3 598 907 de mettre en œuvre un procédé de détection par lequel on compare deux images successives d'une ampoule éclairée en fond noir (l'image n'étant produite que par la lumière diffractée, réfléchie ou déviée des impuretés dans une direction sensiblement perpendiculaire à l'éclairement) produites analogiquement au moyen d'une caméra à balayage. Ne sont pris en compte que les points lumineux produisant une tension analogique supérieure à un certain seuil. Celle-ci est alors mise en forme dans un signal carré ou rectangulaire (fausse numérisation) qui est indépendant de la valeur de dépassement du seuil. Une soustraction de deux images successives si elle n'est pas nulle est significative de la présence d'une impureté. L'un des inconvénients majeurs de cette technique, outre ceux inhérents à la technique du balayage réside dans la présence d'un seuil qui «filtre» la réception avant le traitement du signal proprement dit. Le rôle de ce seuil est d'éliminer le bruit mais comme le fait apparaître le brevet US-A 3 777 169, les signaux utiles sont souvent du même ordre de grandeur que le bruit lui-même. Il est alors conseillé de faire varier ce seuil entre la première et la seconde image pour tenter d'éliminer les bruits et les variations parasites.

Par la présente invention, on se propose de remédier aux inconvénients présentés par les dispositifs et procédés de contrôle connus, et de permettre une détection, qui ne soit perturbée ni par un marquage imprimé sur le récipient, ni par un défaut du récipient lui-même, de sorte que soit rendu possible, de façon automatique, rapide, et avec une grande fiabilité, le tri des récipients contenant au moins une particule indésirable dans un liquide contenu dans ces récipients.

A cet effet, l'invention a pour premier objet un procédé de détection de corps étrangers considérés comme inadmissibles, notamment solides dans un liquide, contenu dans un ou plusieurs récipients transparents, à une certaine lumière, procédé au cours duquel ladite détection a lieu lorsque le ou les récipients sont immobiles et selon lequel:

i) on met le liquide en mouvement et on l'éclaire par un faisceau de cette lumière de sorte que son image, produite par une réflexion, diffraction ou réfraction sensiblement à 90° par rapport à l'axe du faisceau lumineux correspondant sensiblement aux signaux lumineux provenant de toute la surface apparente du liquide, soit formée sur une surface photosensible,

ii) à un premier instant, on procède à l'analyse point par point d'une première image de cette surface, puis

iii) on procède à la mémorisation des résultats de cette analyse;

iv) à un deuxième instant, on analyse une seconde image point par point, puis

v) on soustrait les résultats de la seconde analyse à ceux mémorisés de la première analyse, et enfin

vi) on compare le résultat de la soustraction selon l'étape v) avec au moins un seuil préétabli significatif desdits corps étrangers.

Ledit procédé est caractérisé en ce que l'analyse selon les étapes ii) et iv) consiste à mesurer la grandeur représentative de chacun desdits signaux point par point,

l'étape iii) consiste à mémoriser la grandeur de chacun desdits signaux,

l'étape v) consiste à soustraire point par point respectivement la grandeur d'un desdits signaux de la seconde analyse à celle mémorisée du signal correspondant de la première analyse, en obtenant ainsi des signaux de différence, et l'étape vi) consiste à comparer chacun desdits signaux de différence au/aux seuil/s préétabli/s significatif/s desdits corps étrangers.

Avantageusement, à des intervalles de temps préalablement choisis qui peuvent être, par exemple, de l'ordre de 20 ms, on peut effectuer la mémorisation des résultats de la deuxième analyse et répéter le procédé ainsi défini pour une série de couples d'images prises à des instants premiers et deuxièmes préalablement choisis, une des images pouvant être associée à plusieurs autres images pour former des couples différents.

Grâce au procédé selon l'invention, les défauts liés à la fabrication du récipient et/ou à l'existence d'un marquage sur celui-ci ne sont pas retenus, lors de la comparaison de deux images successives, car ils produisent le même signal lumineux, aux mêmes endroits précisément repérés sur chacune des deux images.

La détection d'une particule circulant dans le liquide contenu dans le récipient analysé s'effectue dès lors avec fiabilité, cette détection étant basée sur les différences de signaux lumineux créés par la ou les particules circulant dans ce liquide, différence qui en outre peut être connue de manière quantitative.

Il est à noter que le ou les seuils de signal lumineux peuvent être choisis au cours d'une opération préalable d'étalonnage, consistant à examiner des récipients contenant des défauts servant d'étalons selon le système de référence pour le contrôle d'un liquide contenu dans un récipient transparent au mode de détection, faisant l'objet de notre demande de brevet FR-A 2 486 241.

Selon une autre caractéristique additionnelle de l'invention, la valeur du signal électrique provoqué par le passage de l'image d'un même défaut sur la surface sensible étant fonction de son temps de passage, qui croît lorsque la rotation du liquide se ralentit, le ou les seuil/s varie/nt en fonction du temps pour tenir compte du ralentissement du mouvement du liquide dans le ou les récipient/s et obtenir un niveau de détection constant desdits corps étrangers.

De préférence, la variation du ou des seuil/s est commandée à des intervalles de temps prédéterminés et s'effectue selon des valeurs croissantes de ce ou ces seuil/s.

Il est bien connu que la rotation du liquide contenu dans le récipient peut entraîner un phénomène de vortex ou une instabilité de la surface de séparation entre le liquide et l'air. Ces phénomènes entraînent des signaux lumineux variables qu'il ne faut pas interpréter comme des défauts. Dans ce but, et selon une autre caratéristique additionnelle de l'invention, les comparaisons deux à deux des images successives s'effectuent sur des zones croissantes depuis l'image du fond du récipient vers l'image de la surface du liquide, de façon à ne pas y inclure le vortex ou l'instabilité mentionnés ci-dessus. Cette mesure permet de commencer le contrôle du récipient très tôt après son blocage. Selon une caractéristique supplémentaire de l'invention, on contrôle simultanément plusieurs récipients adjacents, et la localisation, sur la surface sensible, d'au moins une zone dans laquelle un signal de différence est supérieur audit seuil préétabli commande l'émission d'au moins un signal d'identification du ou des récipients en cause pour l'élimination de ce ou ces récipients.

L'invention a pour second objet un dispositif, destiné à la mise en œuvre du procédé qui vient d'être présenté, qui comprend:

– un système de mise en mouvement et d'immobilisation, subséquent du ou des récipients,
– une source lumineuse délivrant un faisceau de cette lumière pour l'éclairage du ou des récipients,
– un ensemble détecteur constitué par une optique et une surface photosensible, dans lequel l'axe du faisceau lumineux éclairant le ou les récipients est sensiblement perpendiculaire à l'axe optique de l'ensemble détecteur, les signaux lumineux qui proviennent de la surface apparente du ou des récipients, éclairés par le faisceau de lumière touchant la surface photosensible et formant ainsi une image point par point de la surface apparente du liquide sur ladite surface photosensible,
– une mémoire pour mémoriser les données relatives à l'image prise à un certain instant,
– un premier organe de comparaison faisant apparaître par soustraction les données mémorisées de l'image prise à un certain instant et les données d'une image prise à un autre instant,
– et un second organe de comparaison comparant le résultat de la soustraction à au moins un seuil préétabli, significatif desdits corps étrangers.

Selon l'invention, la surface photosensible est constituée par une pluralité de microphotorécepteurs se présentant sous la forme d'une matrice de cellules de réception et de lecture adjacentes, mécaniquement et électriquement délimitées, excitables et lisibles indépendamment les unes des autres, la surface de la matrice étant supérieure à l'image de la surface apparente du liquide contenu dans le ou les récipients, définie par la largeur du ou des récipient/s et la hauteur du liquide dans ce ou ces dernier/s, ladite matrice comportant un circuit opto-électronique intégré, dont la sortie est raccordée par une voie analogique serielle à l'entrée d'un convertisseur analogique-numérique dont la sortie est reliée à l'entrée de la mémoire dont la capacité minimale correspond à la capacité nécessaire pour mémoriser point par point les grandeurs desdits signaux, correspondant à ladite image prise à un instant, tandis que le premier organe de comparaison fait apparaître par soustraction les différences des grandeurs desdits signaux pris par paires et appartenant à deux images prises auxdits deux instants différents, et dont au moins la première a été mémorisée et prise comme référence, et tandis que le second organe de comparaison compare lesdites différences au/aux seuil/s préétabli/s susdit/s.

Toujours selon l'invention, le circuit intégré comportant la matrice des photodétecteurs et ses circuits annexes de transfert et de contrôle peut comporter d'autres dispositifs, tels, par exemple, qu'un convertisseur analogique-numérique; dans ce cas, la sortie est composée de plusieurs voies, une par bit de résolution du convertisseur et une par voie de commande ou de synchronisation.

De préférence, le premier organe de comparaison est un soustracteur, dont la sortie est reliée à l'entrée du second organe de comparaison, agencé sous la forme d'un comparateur à seuil, délivrant un signal de dépassement de seuil à un organe général de commande et recevant au moins une valeur numérique de seuil de ce dernier, qui assure simultanément la synchronisation du soustracteur, du convertisseur analogique-numérique, et du détecteur de seuil, et délivre d'une part à la mémoire des signaux de commande de mise en mémoire et des signaux de commande de lecture de la mémoire et, d'autre part, au mécanisme de tri des récipients, un ordre d'aiguillage pour chaque récipient contrôlé.

De plus, l'organe général de commande assure également la synchronisation d'un organe de commande et de mise en forme des signaux utiles à la matrice de cellules photosensibles.

De plus, un amplificateur analogique à gain variable, est interposé entre la sortie de la matrice de cellules photosensibles et l'entrée du convertisseur analogique-numérique, la variation du gain étant obtenue par commande numérique, à intervalles prédéterminés, à partir de l'organe général de commande, de sorte que le gain soit décroissant à compter de l'immobilisation du ou des récipients à contrôler. Cette variation du gain peut être commandée à des intervalles de temps choisis expérimentalement, et l'amplificateur analogique à gain variable permet de respecter le seuil de détection fixé par le réglage préalable du dispositif avec des récipients contenant des défauts étalonnés, comme mentionnés précédemment.

Dans une forme préférée de réalisation, l'organe général de commande du dispositif est un microprocesseur également connecté à une seconde mémoire, contenant les programmes généraux assurant l'enchaînement des séquences de fonctionnement et permettant l'obtention de la suite des images successives, des mémorisations et des comparaisons des images prises deux à deux à un organe d'entrée des programmes particuliers, permettant notamment l'obtention des valeurs numériques des seuils de comparaison et de la commande numérique du gain variable, à un pupitre de commande, à une imprimante, consignant les conditions de contrôle et les résultats, et enfin au mécanisme de préhension et de manipulation des récipients, auquel le microprocesseur délivre un signal de séquencement des ordres mécaniques. Le microprocesseur peut aussi commander un ou plusieurs autres microprocesseurs auxquels il délègue certaines tâches telles que, par exemple, la commande de l'organe soustracteur, la comparaison au/x seuil/s, la synchronisation du convertisseur analogique-numérique.

Dans une forme préférée de réalisation, la matrice de cellules photosensibles du dispositif selon l'invention est constituée par la surface sensible d'une caméra de télévision à transfert de charge, comportant par exemple 144 lignes de 208 cellules élémentaires photosensibles intégrées sur une pastille de silicium, qui comprend également des circuits de contrôle de transfert et de sortie.

Le ou les récipients contrôlés peuvent être éclairés axialement ou latéralement et la matrice de cellules photosensibles, d'axe optique sensiblement perpendiculaire à l'axe du ou des récipients, reçoit l'image de la surface apparente du liquide formée par un objectif recevant la lumière réfléchie, diffractée, réfractée ou atténuée, par le contenu et les parois du ou des récipients.

En cas de besoin, afin de ne pas détériorer la qualité du produit pharmaceutique contenu dans les récipients, un filtre ultraviolet peut être disposé entre la source de lumière et le ou les récipients.

Le cas échéant, un filtre anti-éblouissement, éliminant les longueurs d'onde qui pourraient nuire à la qualité de la détection, compte-tenu de la nature du matériau utilisé pour les inscriptions sur les récipients, peut être disposé avant ou après les récipients. De même, un filtre placé entre le récipient à contrôler et la source permet de limiter les échauffements nuisibles en éliminant les infrarouges inutiles.

Par ailleurs, un faisceau de fibres optiques peut être utilisé pour conduire la lumière de la source jusqu'à la proximité du ou des récipients.

Le dispositif selon l'invention assure de préférence le contrôle simultané de plusieurs récipients. Si l'un de ceux-ci contient un corps étranger considéré comme inadmissible, un signal est émis à la suite de la comparaison de deux images successives, et ce signal, comportant l'identification du récipient à rejeter par localisation sur la surface sensible de la caméra, est traité par le microprocesseur qui commande l'élimination du récipient jugé défectueux.

Il apparaît donc que le procédé et le dispositif selon l'invention utilisent une méthode de détection par réflexion et diffraction, de sorte que les corps étrangers dans le liquide produisent une augmentation de lumière reçue par la surface photosensible. Pour réaliser cette dernière, on utilise une surface sensible de caméra, constituée d'un circuit intégré comprenant un grand nombre de cellules élémentaires (par exemple 30 000), qui sont cependant chacune bien définie géométriquement, et les signaux de sortie de ces cellules passent tous par une même voie, qui amplifie, numérise et range en mémoire les signaux reçus. Ainsi la surface de chaque micro-cellule élémentaire est non seulement mécaniquement délimitée de façon définitive et stable, mais son emplacement est numériquement repéré et la lumière qu'elle reçoit est numérisée (par exemple de 0 à 255). Cette numérisation systématique permet d'effectuer sans erreur ou instabilité préjudiciables la mise en mémoire des images puis leur traitement (comparaisons entre images et comparaisons des différences à un seuil).

Les images numérisées sont ensuite comparées deux à deux de sorte que la surveillance est quasi continue mais effectuée numériquement. Le principe de détection est qu'il suffit que la différence entre un même point de deux images successives soit supérieure à une certaine valeur de seuil, qui peut être variable en fonction du temps, pour commander l'élimination du récipient contrôlé. C'est donc la différence entre deux signaux issus du même point, qui est mise en évidence, ce qui est très différent du fait que le signal reçu par chaque point de chaque image dépasse ou non un seuil fixé de manière absolue. De plus, le nombre très important des cellules de la surface photosensible permet de contrôler simultanément plusieurs récipients, tout en prenant en compte la quasi totalité de la surface apparente du liquide contenu par chacun. Enfin, la méthode de détection par réflexion et diffraction procure simultanément l'avantage qu'il est également possible de réaliser un contrôle du niveau de remplissage des récipients, par détection de la position de la surface de séparation entre le liquide et l'air, laquelle constitue une zone d'extrême brillance sur toute la largeur du liquide contenu dans le récipient.

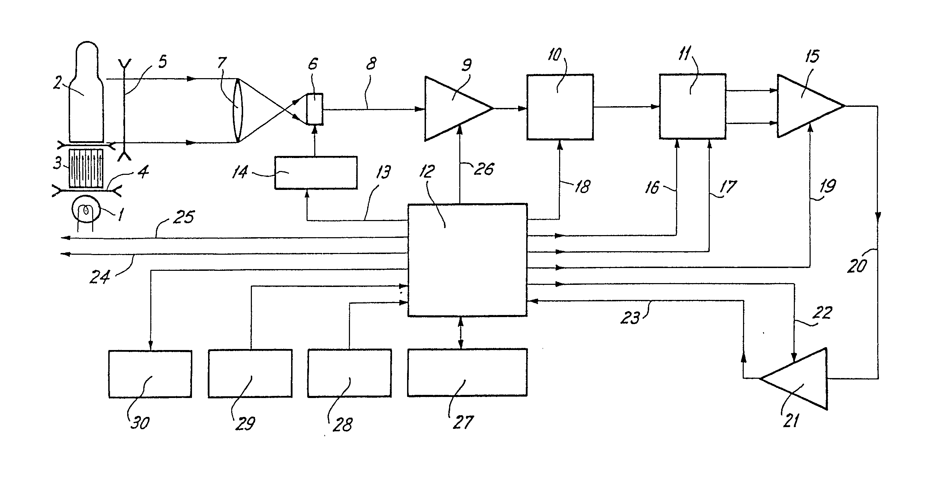

La présente invention sera mieux comprise à l'aide d'un exemple de réalisation, qui sera décrit ci-après, à titre non limitatif, en référence à l'unique figure annexée, représentant de manière symbolique le dispositif utilisé pour la mise en œuvre du procédé selon l'invention.

Le dispositif représenté de manière symbolique sur la figure unique comprend une source de lumière 1, éclairant axialement par en-dessous un groupe de cinq flacons adjacents 2, à contrôler, réalisés en un matériau transparent pour la lumière de la source 1, et contenant un produit pharmaceutique injectable, dans lequel se trouvent éventuellement quelques particules en suspension.

La source de lumière 1 peut comprendre une ampoule basse tension quartz/halogènes, un réflecteur et, le cas échéant, une optique de concentration. Un faisceau de fibres optiques 3 conduit la lumière incidente de la source 1 jusqu'à proximité immédiate du fond de chaque flacon 2, et si utile un filtre 4 éliminant l'ultraviolet est disposé entre la source 1 et ces flacons 2 pour ne pas détériorer le produit injectable, en cas de besoin. Un système optique permettant de rendre pseudocylindrique le faisceau de lumière à l'intérieur de l'ampoule et un diaphragme (tous deux non représentés) réduisant la section lumineuse au diamètre intérieur des flacons 2 peuvent également être prévus entre la sortie des fibres 3 et ces flacons 2 pour diminuer l'éclairement du flacon et dans la lumière parasite émise par les inscriptions et les défauts des récipients. La lumière réfléchie et diffractée par les flacons 2 et leur contenu (liquide et particules en suspension) est filtrée par un filtre anti-éblouissement 5, éliminant les longueurs d'onde pouvant nuire à la qualité de la détection, compte-tenu de la nature du ou des matériaux utilisés pour réaliser les marquages ou inscriptions sur les flacons 2, et l'image de ces flacons 2 est formée sur une surface photosensible 6 par un objectif 7, convenablement choisi, compte-tenu du grandissement souhaité, de la distance entre les flacons 2 et la surface photosensible 6, et de la luminosité, conformément aux règles de l'art.

La surface 6 est constituée par la surface sensible d'une caméra de télévision à transfert de charge, réalisée sous la forme d'un circuit optoélectronique intégré. Cette surface sensible se présente sous la forme d'une matrice de cellules élémentaires de réception et de lecture, adjacentes, physiquement et mécaniquement délimitées et fixes les unes par rapport aux autres, et susceptibles d'être excitées indépendamment les unes des autres. Cette matrice est, par exemple, constituée de 144 lignes de 208 cellules élémentaires photosensibles, soit un total de 29 952 cellules, dont chacune et de forme rectangulaire et mesure 30 microns de long et 28 microns de large, ce qui représente une superficie de $84 \times 10^{-5} mm^2$.

Cette surface photosensible 6, qui peut emporter beaucoup plus de 30 000 cellules, reçoit donc l'image de toute ou presque toute la surface apparente du liquide contenu dans chacun des cinq flacons 2, cette surface apparente étant définie par la largeur d'un flacon 2 et la hauteur du liquide qu'il contient. Au moins 30 000 cellules d'une surface 6 sont ainsi utiles à la réception de l'image d'un seul flacon et au contrôle de la surface apparente du liquide qu'il contient.

Ces cellules permettent l'exploration point par point de cette surface apparente du liquide, pendant que le contenu du flacon est entraîné en mouvement dans le flacon immobile, en raison du mouvement tourbillonnaire donné au liquide par une mise en rotation préalable suivie d'un arrêt brusque du flacon 2, au moyen d'un dispositif mécanique de préhension et de manœuvre des flacons (non représentés), assurant leur transfert, leur rotation, et, en fin de contrôle, leur tri.

Le circuit intégré comprenant la surface photosensible 6 et ses circuits annexes intérieurs est relié par une unique sortie analogique sérielle 8 à l'entrée d'un amplificateur analogique à gain variable 9, dont le rôle sera expliqué ci-après, et lui-même relié par sa sortie à l'entrée d'un convertisseur analogique-numérique 10. Ce dernier alimente une mémoire 11, dont la capacité minimale est dans cet exemple de deux images d'un groupe de cinq flacons 2.

A un instant initial $T_0$, déterminé par un microprocesseur 12 constituant un organe général de commande du dispositif, et transmis par une ligne de synchronisation 13 à un organe 14 de commande et de mise en forme des signaux utiles au circuit intégré de la surface 6, l'ensemble des cellules de cette dernière analyse point par point, l'image de la surface apparente des flacons 2, et le signal lumineux reçu par chaque cellule élémentaire est mis en mémoire 11, après amplification en 9 et numérisation en 10 (sa numérisation sera par exemple un nombre compris entre 0 et 255 selon la nature du signal). De cette façon, une première image de référence est constituée.

A un instant ultérieur $T_1$, la même surface est à nouveau analysée et le signal lumineux reçu par chaque cellule élémentaire à nouveau mémorisé en 11, de la même façon, pour constituer une seconde image.

Les deux images obtenues aux instants $T_0$ et $T_1$ sont ensuite comparées point par point par un soustracteur 15, relié à la sortie de la mémoire 11, laquelle reçoit du microprocesseur 12 ses ordres de commandes respectivement d'entrée et de lecture par des lignes de commandes 16 et 17, tandis que le convertisseur analogique numérique 10 et le soustracteur 15 sont respectivement commandés depuis le microprocesseur 12 par des lignes de synchronisation 18 et 19.

De manière analogue, de nouvelles images sont mémorisées par paire, aux instants $T_{2n}$ et $T_{2n+1}$, à des intervalles de temps préalablement choisis pouvant par exemple être de l'ordre de 20 ms, puis à nouveau comparées.

Selon l'invention, on peut aussi comparer les images des instants $T_{n+1}$, $T_{n+2}$, $T_{n+3}$, etc. . . à l'image prise comme référence à l'instant $T_n$, puis répéter de telles séquences. Il n'est pas obligatoire de mémoriser les images qui ne sont pas utilisées comme référence: elles peuvent, en sortie du convertisseur analogique-numérique, être comparées directement en temps réel à l'image de référence mise en mémoire.

Les signaux correspondant aux éventuelles différences, témoignant de la présence de particules dans le liquide, sont transmis par la ligne 20 de la sortie du soustracteur 15 à l'entrée d'un comparateur à seuil 21.

Il est clair que les défauts liés à la fabrication des flacons ou à la présence d'une inscription sur ces derniers, qui sont des défauts fixes, produisent le même signal lumineux aux mêmes endroits précisément repérés sur chaque image. La comparaison de deux images ne fait donc apparaître aucune différence, et ces défauts ne sont pas retenus.

Par contre, une particule circulant dans le liquide contenu dans l'un des flacons contrôlés est détecté avec fiabilité, grâce aux différences de signaux lumineux créés par cette particule et dans les images successives.

Afin que ne soient détectés que les seuls corps étrangers ou particules solides, considérés comme inadmissibles car d'une taille supérieure à une certaine limite, un seuil significatif de comparaison, correspondant à un seuil significatif de différence de signal lumineux ponctuel, est choisi au cours d'une opération d'étalonnage préalable, consistant en l'examen de flacons identiques à ceux à contrôler, et contenant des défauts étalons, conformément à l'objet de notre demande de brevet français FR-A 2 486 241, relative à un «système de références pour le contrôle d'un liquide contenu dans un récipient transparent au mode de détection».

La valeur numérique du seuil significatif de comparaison choisi est transmise du microprocesseur 12 au comparateur à seul 21 par la ligne 22, et un signal de dépassement de seuil et délivré à la sortie du comparateur 21 et transmis au microprocesseur 12 par la ligne 23, si un signal de différence à son entrée est supérieur au seuil.

Le dispositif assure donc la comparaison des différences entre les signaux de deux images successives au seuil choisi.

Simultanément, le traitement des signaux reçus par le microprocesseur 12 permet la localisation, sur la surface photosensible 6 de celle/s des zones de cette dernière, affectées chacune au contrôle de l'image de la surface apparente du liquide contenu dans un flacon, pour laquelle ou pour lesquelles une différence au moins entre les signaux lumineux ponctuels mémorisés lors de la prise de deux images successives s'est avérée supérieure au seuil.

A la suite de cette localisation, le microprocesseur 12 communique par la ligne 24 un ordre d'aiguillage pour chaque flacon contrôlé à un dispositif mécanique assurant leur tri à la sortie du dispositif de contrôle.

En synchronisme, le microprocesseur 12 pilote également le dispositif mécanique de préhension et de manipulation des flacons 2, que comprend le dispositif de contrôle, pour assurer la mise en mouvement des flacons 2 sur eux-mêmes, et développer le mouvement tourbillonnaire de leur contenu, puis leur brusque immobilisation, et les ordres correspondants sont transmis par une ligne 25 de séquencement des opérations mécaniques.

Comme la rotation du contenu des flacons 2 dans ces derniers peut entraîner un phénomène de vortex ou une instabilité de la surface de séparation entre le liquide et l'air dans chaque flacon 2, la surface photosensible 6 peut recevoir des signaux lumineux variables ne devant pas être interprétés comme témoignant de la présence de particules inadmissibles dans le liquide. Dans ce but, le microprocesseur 12 pilote l'organe 14 de commande et de mise en forme des signaux utiles à la surface photosensible 6, de sorte que des zones croissantes de la surface photosensible 6, depuis la partie de cette dernière recevant l'image du fond des flacons 2 vers la partie recevant l'image de la surface du liquide dans les flacons 2, sont activées, pour permettre la prise d'images successivement comparées, sans y inclure l'image du vortex ou de l'instabilité. Cette mesure permet donc de commencer le contrôle des flacons 2 très tôt après leur immobilisation.

De plus, comme la rotation du contenu des flacons 2 dans ces derniers se ralentit progressivement, le temps de passage de l'image d'un même corps étranger sur une cellule élémentaire de la matrice de la surface sensible 6 augmente. Or la valeur du signal électrique délivrée par cette cellule élémentaire est, entre certaines limites fonction de ce temps de passage, donc également la différence de deux signaux électriques délivrés à des instants successifs. Le dispositif de détection permet de faire varier la valeur retenue comme différence significative entre les signaux électriques provenant de chaque cellule élémentaire, c'est-à-dire le seuil significatif de comparaison, en fonction du temps, afin de maintenir constant le niveau de détection préalablement choisi.

Ceci est obtenu grâce au microprocesseur 12, communiquant par la ligne 22 une valeur numérique croissante dans le temps du seuil significatif de comparaison au comparateur à seuil 21. Mais, pour compléter l'action de cette variation du seuil significatif de comparaison, le microprocesseur 12 pilote de plus par la ligne 26 l'amplificateur analogique à gain variable 9. La valeur du gain de cet amplificateur 9 est choisie par commande numérique, et sa variation est commandée à intervalles de temps choisis expérimentalement, de sorte que le gain prenne des valeurs décroîssssantes dans le temps à partir de l'immobilisation mécanique des flacons 2, à la suite de leur mise en rotation, et que l'amplificateur 9 ainsi piloté permette de respecter le niveau de détection fixé par le réglage préalable du dispositif de détection au moyen de flacons contenant des défauts étalons.

Lorsque le nouvement tourbillonnaire dans les flacons 2 est très atténué, et donc lorsque la zone activée de la surface photosensible 6 correspond à toute la surface de cette dernière, de façon à contrôler toute l'image de la surface apparente du liquide contenu dans les flacons 2, le dispositif de détection permet également de réaliser un contrôle du niveau de remplissage des flacons 2, par détection de la position de la surface de séparation entre le liquide et l'air dans chaque flacon 2, cette surface de séparation constituant une zone de forte brillance sur toute la largeur du liquide contenu dans le flacon 2.

Les programmes généraux assurant l'enchaînement des séquences et permettant notamment l'obtention des déclenchements des mouvements mécaniques des récipients, des prises de vue successives, des mémorisations et des comparaisons d'images sont stockés dans une mémoire 27 connectée au microprocesseur 12, auquel est également connecté un organe 28 d'entrée des programmes particuliers, tel qu'un lecteur enregistreur de supports magnétiques, permettant l'introduction des valeurs du seuil significatif de comparaison du comparateur 21, des valeurs du gain de l'amplificateur 9, des instructions relatives à la sélection des zones croissantes de la surface photosensible 6, en fonction du temps, et à la sélection de la zone maximale de la surface 6 activée en fonction de la taille et du nombre des flacons 2 à contrôler etc. . .

On notera par ailleurs que la surface photosensible peut comporter une pluralité de cellules occultées dont le signal de sortie, interprété par la logique de traitement de l'ensemble constitue une référence de fond noir permettant une correction globale du dispositif en fonction des dérives dues notamment à la température.

Enfin, un pupitre de commande 29 et une imprimante 30, consignant les conditions et les résultats des contrôles, sont reliés au microprocesseur 12.

L'invention n'est pas limitée à la forme de réalisation qui vient d'être décrite. Dans une variante, pour tenir compte du ralentissement de la rotation du liquide dans les flacons 2, l'intervalle de temps séparant deux images mémorisées et comparées peut être augmenté corrélativement, de sorte que l'image de la ou des particules indésirables ait parcouru une distance supérieure à la dimension d'une cellule photo-électrique élémentaire.

**Revendications**

1. Procédé de détection de corps étrangers considérés comme inadmissibles, notamment solides, dans un liquide, contenu dans un ou plusieurs récipients transparents à une certaine lumière, procédé au cours duquel ladite détection a lieu lorsque le ou les récipients sont immobiles et selon lequel:

i) on met le liquide en mouvement et on l'éclaire par un faisceau de cette lumière de sorte que son image produite par une réflexion, diffraction ou réfraction sensiblement à 90° par rapport à l'axe du faisceau lumineux correspondant sensiblement aux signaux lumineux provenant de toute la surface apparente du liquide soit formée sur une surface photo-sensible,

ii) à un premier instant, on procède à l'analyse point par point d'une première image de cette surface, puis

iii) on procède à la mémorisation des résultats de cette analyse;

iv) à un deuxième instant, on analyse une seconde image point par point, puis

v) on soustrait les résultats de la seconde analyse à ceux mémorisés de la première analyse, et enfin

vi) on compare le résultat de la soustraction selon l'étape v) avec au moins un seuil préétabli significatif desdits corps étrangers,

caractérisé en ce que l'analyse selon les étapes ii) et iv) consiste à mesurer la grandeur représentative de chacun desdits signaux point par point, l'étape iii) consiste à mémoriser la grandeur de chacun desdits signaux, l'étape v) consiste à soustraire point par point respectivement la grandeur d'un desdits signaux de la seconde analyse à celle mémorisée du signal correspondant de la première analyse, en obtenant ainsi des signaux de différence, et l'étape vi) consiste à comparer chacun desdits signaux de différence au/aux seuil/s préétabli/s significatifs desdits corps étrangers.

2. Procédé selon la revendication 1, caractérisé en ce que entre l'étape iv) et v) on effectue la mémorisation des résultats de la deuxième analyse et en ce qu'on répète le procédé ainsi défini pour une série de couples d'images prises à des instants premiers et deuxièmes préalablement choisis, une des images pouvant être associée à plusieurs autres images pour former des couples différents.

3. Procédé selon la revendication 1, caractérisé en ce que le ou les seuil/s varie/nt en fonction du temps pour tenir compte du ralentissement du mouvement du liquide dans le ou les récipient/s (2) et obtenir un niveau de détection constant desdits corps étrangers.

4. Procédé selon la revendication 3, caractérisé en ce que la variation du ou des seuil/s est commandée à des intervalles de temps prédéterminés selon des valeurs croissantes de ce ou ces seuil/s.

5. Procédé selon la revendication 2, caractérisé en ce que les images de la série de couples d'images susdite sont effectuées sur des zones croissantes correspondant au début à la partie du fond du récipient pour s'élargir jusqu'à la surface du liquide.

6. Procédé selon la revendication 1, caractérisé en ce que la localisation, sur la surface sensible, d'au moins une zone dans laquelle un desdits signaux de différence est supérieur au seuil préétabli susdit, commande l'émission d'au moins un signal d'identification du ou des récipients (2) en cause pour l'élimination du ou de ces récipients.

7. Dispositif de détection de corps étrangers notamment solides, dans un liquide contenu dans un ou plusieurs récipients transparents à une certaine lumière, pour la mise en œuvre du procédé, selon la revendication 1, comprenant:

– un système de mise en mouvement et d'immobilisation subséquent du ou des récipients,

– une source lumineuse (1) délivrant un faisceau de cette lumière pour l'éclairage du ou des récipients (2),

– un ensemble détecteur constitué par une optique (7) et une surface photosensible (6) dans lequel l'axe du faisceau lumineux éclairant le ou les récipients (2) est sensiblement perpendiculaire à l'axe optique de l'ensemble détecteur (6, 7), les signaux lumineux qui proviennent de la surface apparente du ou des récipients (2), éclairés par le faisceau de lumière touchant la surface photosensible et formant ainsi une image point par point de la surface apparente du liquide sur ladite surface photosensible,

– une mémoire (11) pour mémoriser les données relatives à l'image prise à un certain instant,

– un premier organe de comparaison (15) faisant apparaître par soustraction les données mémorisées de l'image prise à un certain instant et les données d'une image prise à un autre instant,

– et un second organe de comparaison (21) comparant le résultat de la soustraction à au moins un seuil préétabli, significatif desdits corps étrangers,

caractérisé en ce que la surface photosensible (6) est constituée par une pluralité de microphotorécepteurs se présentant sous la forme d'une matrice de cellules de réception et de lecture adjacentes, mécaniquement et électriquement délimitées, excitables et lisibles indépendamment les unes des autres, la surface de la matrice (6) étant supérieure à l'image de la surface apparente du liquide contenu dans le ou les récipients (2), définie par la largeur du ou de l'ensemble des récipients (2) et la hauteur du liquide dans ce ou ces derniers, ladite matrice (6) comportant un circuit opto-électronique intégré, dont la sortie est raccordée par une voie analogique sérielle (8) à l'entrée d'un convertisseur analogique numérique (10) dont la sortie est reliée à l'entrée de la mémoire (11) dont la capacité minimale correspond à la capacité nécessaire pour mémoriser point par point les grandeurs desdits signaux, correspondant à ladite image prise à un instant, en ce que le premier organe de comparaison (15) fait apparaître par soustraction les différences des grandeurs desdits signaux pris par paires et appartenant à deux images prises auxdits deux instants différents, et dont au moins la première a été mémorisée et prise comme référence, et en ce que le second organe de comparaison (21) compare lesdites différences au/aux seuil/s préétabli/s susdit/s.

8. Dispositif selon la revendication 7, caractérisé en ce que le premier organe de comparaison (15) est un soustracteur, dont la sortie est reliée à l'entrée du second organe de comparaison (21), agencé sous la forme d'un comparateur numérique à seuil, délivrant un signal de dépassement de seuil à un organe général de commande (12) et recevant au moins une valeur numérique de seuil de ce dernier, qui assure simultanément la synchronisation du soustracteur (15) et du convertisseur analogique numérique (10), et délivre d'une part à la mémoire (11) des signaux de commande de mise en mémoire et des signaux de commande de lecture de la mémoire et, d'autre part, à un mécanisme de tri des récipients, un ordre d'aiguillage pour chaque récipient (2) contrôlé.

9. Dispositif selon la revendication 8, caractérisé en ce que l'organe général de commande (12) assure de plus la synchronisation d'un organe (14) de commande et de mise en forme des signaux de sortie de la matrice de cellules photosensibles (6).

10. Dispositif selon la revendication 7, caractérisé en ce qu'un amplificateur analogique à gain variable (9) est interposé entre la sortie de la matrice de cellules photosensibles (6) et l'entrée du convertisseur analogique-numérique (10), la variation de gain étant obtenue par commande numérique, à intervalles prédéterminés, à partir de l'organe général de commande (12), de sorte que le gain soit décroissant à compter de l'immobilisation du ou des récipients à contrôler.

11. Dispositif selon la revendication 8, caractérisé en ce que l'organe général de commande (12) est un microprocesseur, également connecté

– à une seconde mémoire (27), contenant les programmes généraux assurant l'enchaînement des séquences de fonctionnement et permettant l'obtention de la suite d'images successives, des mémorisations et des comparaisons successives de la grandeur des signaux correspondant à des images prises deux à deux,
– à un organe d'entrée (28) de programmes particuliers, permettant notamment l'obtention des valeurs numériques de seuils de comparaison et de la commande numérique du gain variable,
– à un pupitre de commande (29),
– à une imprimante (30), consignant les conditions de contrôle et les résultats et
– à un mécanisme de préhension et de manipulation des récipients, auquel le microprocesseur délivre un signal de séquencement des ordres mécaniques.

12. Dispositif selon la revendication 8, caractérisé en ce que la matrice de cellules photosensibles (6) est constituée par la surface sensible d'une caméra de télévision à transfert de charge, ladite surface constituant un élément du circuit opto-électronique intégré, qui comporte également des éléments de contrôle, de transfert et de sortie.

13. Dispositif selon la revendication 8, caractérisé en ce qu'il comprend un filtre anti-éblouissement (5), disposé entre le récipient (2) et la surface photosensible (6) éliminant les longueurs d'onde nuisant à la qualité de la détection en fonction du ou des matériaux utilisés pour la réalisation d'inscriptions sur le ou les récipients (2).

14. Dispositif selon la revendication 7, caractérisé en ce que la surface photosensible comporte une pluralité de cellules de réception occultées fournissant un signal de référence du niveau de noir pour corriger les dérives dues aux variations de température.

**Patentansprüche**

1. Verfahren zur Detektion von, insbesondere festen, Fremdteilchen, die als unzulässig angesehen werden, in einer Flüssigkeit, die in einem oder mehreren transparenten Gefässen enthalten ist, bei einem bestimmten Licht, wobei bei der Durchführung des Verfahrens die erwähnte Detektion immer dann stattfindet, wenn das oder die Gefässe stillgesetzt werden, und gemäss dem:

i) man die Flüssigkeit in Bewegung versetzt und man sie mittels eines Bündels dieses Lichtes derart beleuchtet, dass ihr Bild, das durch eine Reflexion, Beugung oder Brechung um im wesentlichen 90° in bezug auf die Achse des Lichtbündels erzeugt wird, das im wesentlichen den von der gesamten sichtbaren Fläche der Flüssigkeit ausgehenden Lichtsignalen entspricht, auf einer lichtempfindlichen Fläche dargestellt wird,

ii) man zu einem ersten Zeitpunkt eine punktweise Analyse eines ersten Bildes dieser Fläche durchführt, dann

iii) man die Speicherung der Ergebnisse dieser Analyse durchführt;

iv) man zu einem zweiten Zeitpunkt ein zweites Bild punktweise analysiert, dann

v) man die Ergebnisse der zweiten Analyse von den gespeicherten der ersten Analyse subtrahiert und schliesslich

vi) man das Ergebnis der Subtraktion gemäss dem Schritt v) mit mindestens einer voreingestellten Schwelle vergleicht, die die genannten Fremdteilchen kennzeichnet,
dadurch gekennzeichnet, dass die Analyse gemäss den Schritten ii) und iv) darin besteht, die kennzeichnende Grösse jedes der genannten Signale punktweise zu messen, der Schritt iii) darin besteht, die Grösse jedes der genannten Signale zu speichern, der Schritt v) darin besteht, jeweils die Grösse eines der Signale der zweiten Analyse von der gespeicherten des entsprechenden Signals der ersten Analyse punktweise zu subtrahieren, um auf diese Weise die Differenzsignale zu erhalten, und der Schritt vi) darin besteht, jedes der genannten Differenzsignale mit der/den voreingestellten Schwelle/n zu vergleichen, die die genannten Fremdteilchen kennzeichnen.

2. Verfahren nach Anspruch 1, dadurch gekennzeichnet, dass man zwischen den Schritten iv) und v) die Speicherung der Ergebnisse der zweiten Analyse durchführt und dass man das auf diese Weise festgelegte Verfahren bei einer Folge von Bildpaaren wiederholt, die zu zuvor ausgewählten ersten und zweiten Zeitpunkten aufgenommen wurden, wobei eines der Bilder mehreren anderen Bildern zugeordnet werden kann, um die verschiedenen Paare zu bilden.

3. Verfahren nach Anspruch 1, dadurch gekennzeichnet, dass sich die Schwelle oder die Schwellen als Funktion der Zeit verändert/n, um die Verlangsamung der Bewegung der Flüssigkeit in dem oder den Gefäss/en (2) zu berücksichtigen und ein konstantes Mass für die Erkennung der genannten Fremdteilchen zu erhalten.

4. Verfahren nach Anspruch 3, dadurch gekennzeichnet, dass die Veränderung der Schwelle oder der Schwellen zu vorgegebenen Zeitintervallen gemäss den zunehmenden Werten der Schwelle oder der Schwellen gesteuert wird.

5. Verfahren nach Anspruch 2, dadurch gekennzeichnet, dass die Bilder der obengenannten Folge von Bildpaaren in grösser werdenden Bereichen aufgenommen werden, die am Anfang dem Bodenteil des Gefässes entsprechen, um sich bis zur Oberfläche der Flüssigkeit zu erweitern.

6. Verfahren nach Anspruch 1, dadurch gekennzeichnet, dass die Lokalisierung auf der empfindlichen Fläche mit mindestens einem Bereich, in dem die genannten Differenzsignale grösser sind als die obengenannte voreingestellte Schwelle, das Aussenden mindestens eines Identifikationssignals des oder der fraglichen Gefässe (2) für die Aussonderung des oder dieser Gefässe steuert.

7. Einrichtung zur Detektion von, insbesondere festen, Fremdteilchen in einer Flüssigkeit, die in einem oder mehreren transparenten Gefässen enthalten ist, bei einem bestimmten Licht, zum Durchführen des Verfahrens nach Anspruch 1, enthaltend:

- ein System zum Inbewegungversetzen und zum nachfolgenden Stillsetzen des oder der Gefässe,
- eine Lichtquelle (1), die ein Bündel dieses Lichtes zum Beleuchten des oder der Gefässe (2) liefert,
- eine Detektoreinheit, die aus einer Optik (7) und einer lichtempfindlichen Fläche (6) gebildet wird, in der die Achse des Lichtbündels, das das oder die Gefässe (2) beleuchtet, im wesentlichen senkrecht zur optischen Achse der Detektoreinheit (6, 7) ist und bei der die Lichtsignale, die von der sichtbaren Fläche des oder der durch das Lichtbündel beleuchteten Gefässe (2) ausgehen, auf der lichtempfindlichen Fläche auftreffen und damit punktweise ein Bild von der sichtbaren Fläche der Flüssigkeit auf der genannten lichtempfindlichen Fläche bilden,
- einen Speicher (11) zum Speichern der Daten, die einem zu einem bestimmten Zeitpunkt aufgenommenen Bild zugeordnet sind,
- ein erstes Vergleichsglied (15), das durch Subtraktion die gespeicherten Daten des zu einem bestimmten Zeitpunkt aufgenommenen Bildes und die Daten des zu einem anderen Zeitpunkt aufgenommenen Bildes verarbeitet,
- und ein zweites Vergleichsglied (21), das das Ergebnis der Subtraktion mit einer oder mehreren voreingestellten Schwellen vergleicht, die die genannten Fremdteilchen kennzeichnen, dadurch gekennzeichnet, dass die lichtempfindliche Fläche (6) aus einer Mehrzahl von Mikrolichtempfängern besteht, die sich in der Form einer Matrix von Empfangszellen und benachbarten Auslesezellen darstellen, die mechanisch und elektrisch begrenzt sind und die unabhängig voneinander erregbar und auslesbar sind, wobei die Fläche der Matrix (6) grösser ist als das Bild der sichtbaren Fläche der in dem oder den Gefässen (2) enthaltenen Flüssigkeit, die durch die Breite des oder der Gesamtheit der Gefässe (2) und den Flüssigkeitsstand in dem oder den letzteren definiert ist, wobei die genannte Matrix (6) einen integrierten elektro-optischen Schaltkreis enthält, dessen Ausgang über eine serielle analoge Verbindung (8) mit dem Eingang eines Analog/Digital-Umsetzers (10) verbunden ist, dessen Ausgang mit dem Eingang des Speichers (11) verbunden ist, dessen minimale Kapazität derjenigen Kapazität entspricht, die notwendig ist, um punktweise die Grösse der genannten Signale zu speichern, die dem zu einem Zeitpunkt aufgenommenen genannten Bild entsprechen, dass das erste Vergleichsglied (15) durch Subtraktion die Differenzen der Grössen der genannten Signale verarbeitet, die paarweise erfasst wurden und die zwei Bildern zugehören, die zu den genannten zwei verschiedenen Zeitpunkten aufgenommen wurden und von denen mindestens das erste gespeichert wurde und als Bezugsbild genommen wurde, und dass das zweite Vergleichsglied (21) die genannten Differenzen mit der/den obengenannten Schwelle/n vergleicht.

8. Einrichtung nach Anspruch 7, dadurch gekennzeichnet, dass das erste Vergleichsglied (15) ein Subtrahierer ist, dessen Ausgang mit dem Eingang des zweiten Vergleichsglieds (21) verbunden ist, das als ein digitaler Vergleicher mit einer Schwelle ausgebildet ist, der beim Überschreiten der Schwelle ein Signal an eine Hauptsteuereinheit (12) abgibt und der mindestens einen digitalen Schwellenwert von letzterer erhält, die gleichzeitig die Synchronisation des Subtrahierers (15) und des Analog/Digital-Umsetzers (10) sicherstellt und die einerseits an den Speicher (11) Befehlssignale für das Eingeben in den Speicher und Befehlssignale für das Lesen aus dem Speicher und andererseits an einen Sortiermechanismus für die Gefässe einen Befehl zur Weichenstellung für jedes überprüfte Gefäss (2) liefert.

9. Einrichtung nach Anspruch 8, dadurch gekennzeichnet, dass die Hauptsteuereinheit (12) darüber hinaus die Synchronisation eines Steuerteils (14) und eines Teils zum Umformen der Ausgangssignale der Matrix aus lichtempflindlichen Zellen (6) sicherstellt.

10. Einrichtung nach Anspruch 7, dadurch gekennzeichnet, dass ein Analogverstärker (9) mit veränderbarer Verstärkung zwischen dem Ausgang der Matrix mit den lichtempfindlichen Zellen (6) und dem Eingang des Analog/Digital-Umsetzers (10) eingefügt ist, wobei die Veränderung der Verstärkung in vorgegebenen Zeitabständen durch einen von der Hauptsteuereinheit abgegebenen numerischen Befehl derart bewerkstelligt wird, dass die Verstärkung von dem Stillsetzen des oder der zu überprüfenden Gefässe an verkleinert wird.

11. Einrichtung nach Anspruch 8, dadurch gekennzeichnet, dass die Hauptsteuereinheit (12) ein Mikroprozessor ist, der auch verbunden ist

- mit einem zweiten Speicher (27), der die Hauptprogramme enthält, die die Verkettung der Funktionsfolgen sicherstellt und es ermöglicht, nacheinander die aufeinanderfolgenden Bilder, die Speicherungen und die aufeinanderfolgenden Vergleiche der Grösse der Signale zu erreichen, die den jeweils paarweise genommenen Bildern entsprechen,
- mit einer Eingabeeinheit (28) für besondere Programme, die es insbesondere erlaubt, die numerischen Schwellenwerte beim Vergleichen und den numerischen Befehl für die veränderbare Verstärkung zu erhalten,
- mit einer Befehlskonsole (29),
- mit einem Drucker (30), der die Bedingungen bei der Überprüfung und die Ergebnisse aufzeichnet und
- mit einem Mechanismus zum Erfassen und Handhaben der Gefässe, an den der Mikroprozessor ein Signal für die Aufeinanderfolge der mechanischen Befehle liefert.

12. Einrichtung nach Anspruch 8, dadurch gekennzeichnet, dass die Matrix mit den lichtempfindlichen Zellen (6) aus der empfindlichen Fläche einer Fernsehkamera mit Ladungskopplung gebildet wird, wobei die genannte Fläche einen integrierten opto-elektronischen Schaltkreis aufweist, der auch Überwachungs-, Übertragungs- und Ausgangselemente enthält.

13. Einrichtung nach Anspruch 8, dadurch gekennzeichnet, dass sie ein zwischen dem Gefäss (2) und der lichtempfindlichen Fläche (6) angeordnetes Abblendfilter (5) enthält, das die Wellenlängen beseitigt, die für die Qualität der Erkennung in Abhängigkeit von dem oder den verwendeten Materialien für die Realisierung der Beschriftungen auf dem oder den Gefässen (2) schädlich ist.

14. Einrichtung nach Anspruch 7, dadurch gekennzeichnet, dass die lichtempfindliche Fläche eine Mehrzahl von abgedunkelten Empfangszellen aufweist, die ein Bezugssignal mit dem Schwarzpegel liefern, um die Abweichungen infolge der Veränderungen der Temperatur zu korrigieren.

**Claims**

1. A method for detecting foreign bodies considered inadmissable, in particular solids, in a liquid contained in one or more containers transparent to a certain light, in the course of which method said detection takes place when the container or containers are motionless, and in which:

i) The liquid is put into motion and illuminated by a beam of said light so that its image, produced by reflection, diffraction or refraction at approximately 90° relative to the axis of the light beam corresponding approximately to the light signals issuing from the entire visible surface of the liquid, is formed on a photosensitive surface,

ii) at a first instant, a first image of this surface is analysed point by point, then

iii) the results of this analysis are stored;

iv) at a second instant, a second image is analysed point by point, then

v) the results of the second analysis are subtracted from those of the first, and finally

vi) the result of the subtraction in v) is compared with at least one predetermined significant threshold of said foreign bodies, characterised in that the analysis in stages ii) and iv) consists of measuring the representative magnitude of each of the said signals point by point, stage iii) consists of storing the magnitude of each of said signals, stage v) consists of subtracting, point by point, the magnitude of one of the signals from the second analysis from that of the corresponding signal from the first analysis, thus obtaining difference signals, and stage vi) consists of comparing each of the said difference signals with the predetermined significant threshold of said foreign bodies.

2. A method according to Claim 1, characterised in that the results of the second analysis are stored between stages iv) and v), and that the process is repeated for a series of image pairs taken at the first and second instants already selected, one of the images being associable with several other images to form different pairs.

3. A method according to Claim 1, characterised in that the threshold or thresholds vary as a function of time in order to take into account the slowing down of the movement of the liquid in the container or containers (2) and to achieve a constant level of detection of said foreign bodies.

4. A method according to Claim 3, characterised in that the variation in the threshold or thresholds is controlled at predetermined time intervals according to the rising threshold value or values.

5. A method according to Claim 2, characterised in that the images in the above series of image pairs are formed on increasing zones corresponding initially to the base part of the container and increasing up to the surface of the liquid.

6. A method according to Claim 1, characterised in that the positioning on the sensitive surface of at least one zone in which one of the said difference signals exceeds the above predetermined threshold controls the emission of at least one identification signal from the container or containers (2) to eliminate said container or containers.

7. A device for detecting foreign bodies, in particular solids, in a liquid contained in one or more containers transparent to a certain light, for implementing the method according to Claim 1, comprising:

– a system for putting into motion and subsequent immobilisation of the container or containers,

– a light source (1) delivering a beam of light to illuminate the container or containers (2),

– a detecting assembly comprising a lens (7) and a photosensitive surface (6) in which assembly the axis of the beam of light illuminating the container or containers (2) is approximately perpendicular to the optical axis of the detecting assembly (6, 7), the light signals which come from the visible surface of the container or containers (2), illuminated by the beam of light, touching the photosensitive surface and thus forming an image point by point of the visible surface of the liquid on said photosensitive surface,

– a store (11) for storing the date relative to the image taken at a particular instant,

– a first comparing element (15) showing the stored data of the image taken at one instant and the data of an image taken at another instant by subtraction,

– and a second comparing element (21) comparing the result of the subtraction with at least one predetermined threshold significant for said foreign bodies, characterised in that the photosensitive surface (6) comprises a plurality of microphotoreceivers in the form of a matrix of adjacent receiver and read cells, which are mechanically and electrically delimited, and which are excitable and readable independently of one another, the surface of the matrix (6) being above the image of the visible surface of the liquid contained in the container or containers (2), which surface is defined by the width of the container or assembly of containers (2) and the height of the liquid in said container or containers, said matrix comprising an integrated electronic circuit, the output of which is connected by a serial analogue channel (8) to the input of an analog-to-digital converter (10), the output of which is connected to the input of the store (11), the minimum capacity of which corresponds to the capacity required for storing the magnitude of said signals point by point, corresponding to said image taken at one instant, and in that the first comparing element (15) shows by subtraction the difference between the magnitude of said signals taken in pairs and belonging to the two images formed at said two different instants, and of which at least the first has been stored and used as a reference, and in that the second comparing element (21) compares said differences with the above predetermined threshold or thresholds.

8. A device according to Claim 7, characterised in that the first comparing element (15) is a subtractor, the output of which is connected to the input of the second comparing element (21) in the form of a numerical threshold comparator delivering a threshold overshoot signal to a general control element (12) and receiving at least one numerical threshold value from the latter, which simultaneously ensures the synchronization of the subtractor (15) and the analog-to-digital converter (10), and, on the one hand, delivers storing control signals and store reading signals to the store and, on the other, sends a switching instruction for each container (2) checked to a container sorting mechanism.

9. A device according to Claim 8, characterised in that the general control element (12) further ensures synchronization of an organ (14) for controlling and formatting the output signals of the matrix of photosensitive cells (6).

10. A device according to Claim 7, characterised in that an analog variable-gain amplifier (9) is interposed between the output of the photosensitive cell matrix (6) and the input of the analog-to-digital converter (10), the variation in gain being obtained by numeric control, at predetermined intervals, starting with the general control element (12), such that the gain decreases with effect from the immobilisation of the container or containers to be checked.

11. A device according to Claim 8, characterised in that the general control element (12) is a microprocessor, connected to
   - a second store (27) containing general programs ensuring the concatenation of the operation sequences and enabling the attainment of a series of successive images, storage and successive comparison of the magnitude of the signals corresponding to the images taken in pairs,
   - an input element (28) for special programs enabling in particular the acquisition of comparative numerical threshold values and the digital control of variable gain,
   - a control desk (29),
   - a printer (30) for recording the control conditions and results, and
   - a mechanism for holding and manipulating the containers, to which the microprocessor delivers a signal for the sequence of mechanical orders.

12. A device according to Claim 8, characterised in that the matrix of photosensitive cells (6) comprises the sensitive surface of a charge-transfer television camera, said surface forming a part of the integrated opto-electronic circuit, which includes control, transfer and output elements.

13. A device according to Claim 8, characterised in that it comprises an anti-glare filter (5) disposed between the container (2) and the photosensitive surface (6), thus eliminating the wavelengths from the material or materials used to make inscriptions on the container or containers (2) which would impair the quality of the detection.

14. A device according to Claim 7, characterised in that the photosensitive surface comprises a plurality of occulted receiver cells providing a reference signal for the level of blackness in order to correct the drifts caused by variations in temperature.